(19) Europäisches Patentamt
European Patent Office
Office européen des brevets

(11) EP 2 532 647 B1

(12) # FASCICULE DE BREVET EUROPEEN

(45) Date de publication et mention de la délivrance du brevet:
**28.09.2016 Bulletin 2016/39**

(21) Numéro de dépôt: **12171231.9**

(22) Date de dépôt: **08.06.2012**

(51) Int Cl.:
***C07C 231/12*** *(2006.01)* ***C07C 233/18*** *(2006.01)*
***A61K 31/165*** *(2006.01)* ***A61P 25/16*** *(2006.01)*
***A61P 25/18*** *(2006.01)* ***A61P 25/28*** *(2006.01)*
***A61P 3/00*** *(2006.01)*

(54) **Nouveaux co-cristaux d'agomelatin.**

Neue co-kristalle von agomelatin

New co-crystals of agomelatin.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **09.06.2011 FR 1101766**
**25.08.2011 CN 201110245039**

(43) Date de publication de la demande:
**12.12.2012 Bulletin 2012/50**

(73) Titulaire: **Les Laboratoires Servier**
**92284 Suresnes Cedex (FR)**

(72) Inventeurs:
- **Letellier, Philippe**
**45000 Orléans (FR)**
- **Lynch, Michael**
**45140 Saint Jean de la Ruelle (FR)**
- **Pean, Jean-Manuel**
**45000 ORLEANS (FR)**

(56) Documents cités:
**EP-A1- 2 517 700** **WO-A1-2005/002562**
**WO-A2-2012/046253**

- **SAI-LI ZHENG ET AL: "Structures of Polymorphic Agomelatine and Its Cocrystals with Acetic Acid and Ethylene Glycol", CRYSTAL GROWTH & DESIGN, ACS, WASHINGTON, DC, US, vol. 11, 2 février 2011 (2011-02-02), pages 466-471, XP002658583, ISSN: 1528-7483, DOI: 10.1021/CG101234P [extrait le 2011-01-06]**
- **REMENAR J F ET AL: "Crystal engineering of novel cocrystals of a tiazole drug with 1,4-dicarboxylic acids", JOURNAL OF THE AMERICAN CHEMICAL SOCIETY, AMERICAN CHEMICAL SOCIETY, WASHINGTON, DC; US, vol. 125, 1 juin 2003 (2003-06-01), pages 8456-8457, XP002973472, ISSN: 0002-7863, DOI: 10.1021/JA035776P**
- **TRASK A V ET AL: "Physical stability enhancement of theophylline via cocrystallization", INTERNATIONAL JOURNAL OF PHARMACEUTICS, ELSEVIER BV, NL, vol. 320, no. 1-2, 31 août 2006 (2006-08-31), pages 114-123, XP025113479, ISSN: 0378-5173, DOI: 10.1016/J.IJPHARM.2006.04.018 [extrait le 2006-08-31]**
- **DANIEL P MCNAMARA ET AL: "Use of a Glutaric Acid Cocrystal to Improve Oral Bioavailability of a Low Solubility API", PHARMACEUTICAL RESEARCH, KLUWER ACADEMIC PUBLISHERS-PLENUM PUBLISHERS, NL, vol. 23, no. 8, 11 juillet 2006 (2006-07-11), pages 1888-1897, XP019405182, ISSN: 1573-904X, DOI: 10.1007/S11095-006-9032-3**
- **MARTIN VIERTELHAUS ET AL: "Piracetam Co-Crystals with OH-Group Functionalized Carboxylic Acids", CRYSTAL GROWTH & DESIGN, vol. 9, no. 5, 6 mai 2009 (2009-05-06), pages 2220-2228, XP055011940, ISSN: 1528-7483, DOI: 10.1021/cg800942n**
- **NATE SCHULTHEISS ET AL: "Pharmaceutical Cocrystals and Their Physicochemical Properties", CRYSTAL GROWTH & DESIGN, vol. 9, no. 6, 3 juin 2009 (2009-06-03), pages 2950-2967, XP055011939, ISSN: 1528-7483, DOI: 10.1021/cg900129f**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **M.C. ETTER ET AL: "Solid-state nucleophilic aromatic substitution reaction of a carboxylic acid cocrystal", TETRAHEDRON LETTERS, vol. 30, no. 28, 1 janvier 1989 (1989-01-01), pages 3617-3620, XP055011861, ISSN: 0040-4039, DOI: 10.1016/S0040-4039(01)80463-9**

Remarques:

Le dossier contient des informations techniques présentées postérieurement au dépôt de la demande et ne figurant pas dans le présent fascicule.

## Description

La présente invention concerne de nouveaux co-cristaux d'agomélatine ou *N*-[2-(7-méthoxy-1-naphtyl)éthyl] acétamide de formule (I) :

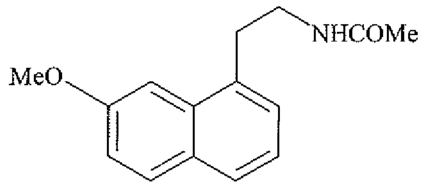

(I)

leur procédé de préparation ainsi que les compositions pharmaceutiques qui les contiennent.

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-$HT_{2C}$. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte tenu de l'intérêt pharmaceutique de ce composé, de nombreux travaux de recherche ont été menés et ont permis d'isoler différentes formes polymorphiques, présentant divers avantages, notamment de pureté, de stabilité, de reproductibilité, de formulation... permettant un stockage prolongé sans conditions particulières de température, de lumière, d'humidité ou de taux d'oxygène.

Par ailleurs, comme pour tout principe actif destiné à être administré à l'homme, il est très important de pouvoir maîtriser sa vitesse de dissolution afin de favoriser une diffusion rapide ou au contraire lente.

La demanderesse a présentement élaboré de nouveaux co-cristaux d'agomélatine permettant de modifier la vitesse de dissolution du principe actif. Les co-cristaux selon l'invention présentent une vitesse de dissolution accélérée ou ralentie comparée à la forme disponible sur le marché décrite dans le brevet EP1564202 et commercialisée sous la marque Valdoxan®. Ces nouveaux co-cristaux à profil de dissolution modifié permettent ainsi d'envisager de nouvelles formulations en fonction de l'utilisation souhaitée.

On trouve dans l'art antérieur WO2005/00256 une revendication concernant un co-cristal d'agomélatine avec l'acide tartrique. Cet art antérieur ne fait état d'aucune caractérisation ni d'aucun mode d'obtention.

Sai-Li Zheng et al. (Crystal Growth & Design, 2011, 11, 466-471) décrit un co-cristal d'agomélatine obtenu avec l'acide acétique, mais l'étude de ce co-cristal a montré qu'il est très instable et correspond à un solvate très labile.

Un co-cristal est un complexe cristallin composé d'au moins deux molécules neutres liées ensemble dans un réseau cristallin par des interactions non covalentes. La principale différence entre les solvates et les co-cristaux est liée à l'état physique des composants purs : si l'un des constituants est liquide à température ambiante, alors le complexe moléculaire est un solvate ; si tous les composants sont solides à température ambiante, alors le complexe est désigné sous le terme de co-cristal. La différence majeure entre solvate et co-cristal est la stabilité bien supérieure du co-cristal par rapport au solvate. Un co-cristal est caractérisé par un procédé d'obtention et une structure tridimensionnelle ordonnée mise en évidence par des diagrammes de diffraction aux rayons X par exemple. Il n'est pas possible *a priori* de savoir si deux constituants donnés vont pouvoir former un co-cristal avec une structure tridimensionnelle particulière out simplement donner lieu à une juxtaposition des deux poudres. Cette structure tridimensionnelle particulière est directement reliée à la vitesse de dissolution de l'entité ainsi formée.

Plus particulièrement l'invention concerne de nouveaux co-cristaux constitués d'agomélatine d'une part et d'un acide organique d'autre part. Les co-cristaux selon l'invention contiennent des acides organiques présentant un état solide à température ambiante.

Les acides organiques selon l'invention sont des acides linéaires contenant de 2 à 10 atomes de carbone. Ils présentent une ou plusieurs fonctions acide COOH, et plus préférentiellement une, deux ou trois fonctions acides. Ils peuvent également présenter, en plus de leur(s) fonction(s) acide(s) une ou plusieurs fonctions cétone, une ou plusieurs fonctions hydroxy, une ou plusieurs insaturations.

Les acides organiques constituants des co-cristaux selon l'invention, sont l'acide parahydroxybenzoïque, l'acide gallique, l'acide malonique, l'acide glutarique, l'acide glycolique, l'acide cétoglutarique...

La proportion d'acide organique utilisée par rapport à l'agomélatine varie de 0,25 à 4 équivalents molaires, et préférentiellement de 0,5 à 2 équivalents molaires.

Plus particulièrement, l'invention concerne les co-cristaux suivants : agomélatine/acide parahydroxybenzoïque (2/1) et (1/2) ; agomélatine/acide gallique (2/1) ; agomélatine/acide malonique (1/1) ; agomélatine/acide glutarique (1/1) ; agomélatine/acide glycolique (1/1) ; agomélatine/acide cétoglutarique (1/1).

L'invention concerne également un procédé d'obtention des co-cristaux d'agomélatine et d'acides organiques, dans lequel :

- les deux constituants sont mélangés au sein d'un solvant organique dans les proportions désirées (1 équivalent d'agomélatine pour 0,25 à 4 équivalents molaires d'acide organique) ;
- la solution obtenue est agitée et optionnellement chauffée à une température au plus égale à la température d'ébullition du solvant choisi ;
- le milieu est refroidi sous agitation et le co-cristal précipite naturellement ou précipite après reprise dans un deuxième solvant ;
- le précipité obtenu est filtré et séché.

Dans le procédé selon l'invention, le solvant utilisé est préférentiellement un alcool comme par exemple le méthanol ou le *tert*-butanol ; un éther comme par exemple l'éther diisopropylique ou l'éther de méthyle et de *tert*-butyle ; ou un hydrocarbure aromatique comme le toluène par exemple. Lorsqu'un deuxième solvant est utilisé pour favoriser la précipitation du co-cristal, le benzonitrile est avantageusement choisi.

Un procédé alternatif consiste à co-broyer les deux constituants du co-cristal. Préférentiellement le co-broyage est effectué dans une jarre en acier. Une variante de ce procédé consiste à rajouter un solvant organique lors du broyage ; dans ce cas le co-cristal obtenu est ensuite séché. Parmi les solvants utilisés on citera plus particulièrement les alcools comme l'éthanol par exemple, ou les éthers comme l'éther diisopropylique par exemple.
Avantageusement, le broyage est réalisé avec des billes en inox. Le broyage est réalisé au moyen de vibrations, préférentiellement des vibrations avec une fréquence allant de 20 à 30 Hz. Les vibrations sont appliquées pendant une durée pouvant aller de 15 minutes à 3 heures.

Un autre procédé alternatif consiste à mélanger deux solutions contenant chacun des constituants et à congeler rapidement à très basse température le mélange obtenu, puis à sécher à cette même basse température le co-cristal ainsi obtenu. Avantageusement, les deux constituants sont mélangés au sein d'un solvant organique ou hydro-organique. De façon préférée, la congélation et le séchage sont effectués entre -40°C et -60°C, et plus préférentiellement à -40°C.

Un autre procédé avantageux selon l'invention consiste à mélanger les poudres d'agomélatine et de l'acide considéré dans un mélangeur, puis à l'extruder par extrusion bi-vis sans filière pour obtenir un grain solide directement en sortie d'extrudeuse. De préférence, le profil de vis utilisé est un profil à fort cisaillement, avec optionnellement l'utilisation d'éléments malaxeurs permettant d'améliorer la surface de contact entre les constituants. Le paramètre L/D de la vis peut varier entre 10 et 40 et la vitesse de rotation entre 10 et 200 tr/min. La température utilisée varie de 40 à 100 °C.

Dans les procédés de préparation des co-cristaux selon l'invention, on peut utiliser le composé de formule (I) obtenu par n'importe quel procédé, notamment celui décrit dans EP1564202(A1).

Les co-cristaux selon l'invention montrent des propriétés tout à fait intéressantes en termes de stabilité et de dissolution, deux paramètres essentiels dans l'industrie pharmaceutique. La dissolution de principes actifs est un phénomène important qui peut déterminer la vitesse d'adsorption de ceux-ci dans le corps humain. C'est une étape importante du processus de la libération, qui détermine dans une large mesure l'activité d'un médicament. En effet, pour traverser les membranes biologiques ou pour être absorbé, le principe actif doit être dispersé, à l'état moléculaire en milieux aqueux (c'est-à-dire dissous) au site d'absorption. La vitesse de dissolution du principe actif est fonction de ses caractéristiques physico-chimiques ainsi que des conditions du milieu d'absorption. Il est ainsi important de pouvoir disposer de formes présentant une vitesse de dissolution modifiée du principe actif, permettant d'obtenir une dissolution plus ou moins rapide du principe actif en fonction de l'utilisation souhaitée : une forme présentant une dissolution améliorée pour une utilisation dans des formulations à libération immédiate, et une forme présentant une dissolution moins rapide pour une utilisation dans des formulations retard ou à libération différée.
Les co-cristaux selon l'invention répondent à ce besoin puisqu'il est possible de modifier la vitesse de dissolution de l'agomélatine et favoriser ou diminuer sa dissolution d'un facteur allant jusqu'à 2 par rapport à la forme actuellement commercialisée sous la spécialité Valdoxan®. En particulier, les co-cristaux selon l'invention permettent une modification de la vitesse de dissolution du principe actif par rapport à la vitesse de dissolution de la forme actuellement commercialisée sous la spécialité Valdoxan® d'au moins 25% en conditions neutre (pH 6,8) ou acide (HCl 0,01N). Il est ainsi possible d'utiliser les co-cristaux selon l'invention dans l'élaboration de formes pharmaceutiques à libération immédiate dans lesquelles la vitesse de dissolution est améliorée par rapport à la forme actuellement disponible sur le marché, ainsi que dans des formes à libération différée dans lesquelles la vitesse de dissolution est retardée.

Les formes pharmaceutiques contenant les co-cristaux selon l'invention seront utilisées pour leur activité sur le système nerveux central ainsi que sur la microcirculation, dans le traitement du stress, des troubles du sommeil, des

troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, les co-cristaux selon l'invention pourront être utilisés dans les dysfonctionnements sexuels, en tant qu'inhibiteurs de l'ovulation, immunomodulateurs et dans le traitement des cancers.

Les co-cristaux selon l'invention seront utilisés de préférence dans les traitements de la dépression majeure, des dépressions saisonnières, des troubles du sommeil, des troubles de l'anxiété, des troubles de l'humeur, des pathologies cardiovasculaires, des pathologies du système digestif, des insomnies et fatigues dues aux décalages horaires, des troubles de l'appétit et de l'obésité.

L'invention s'étend aussi aux compositions pharmaceutiques renfermant comme principe actif un co-cristal selon l'invention avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour d'agomélatine en une ou plusieurs prises.

Les exemples ci-dessous illustrent l'invention, mais ne la limitent en aucune façon.

**<u>Exemple 1</u> : Co-cristal Agomélatine/Acide gallique (2/1)**

Une solution de 300,6 mg de *N*-[2-(7-Méthoxy-1-naphtyl)éthyl]acétamide dans 15 ml de tert-butanol est ajoutée lentement à une solution de 106 mg d'acide gallique dans 35 ml d'eau dans un ballon de 250 ml. Le milieu est agité 10 minutes puis la solution est congelée à -40°C et séchée à cette même température pendant 2 jours pour conduire au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en °±0,2), et d'intensité relative (exprimée en pourcentage par rapport à la raie la plus intense) :

| 2-Theta (°) exp. | d (Å) exp. | Intensité (%) |
|---|---|---|
| 7,4888 | 11,8051 | 13,8 |
| 9,9347 | 8,90352 | 14,42 |
| 12,456 | 7,10638 | 9,11 |
| 12,7479 | 6,9443 | 14,08 |
| 14,0965 | 6,28286 | 5,63 |
| 14,4701 | 6,12146 | 20,24 |
| 16,7302 | 5,29926 | 14,01 |
| 16,829 | 5,26837 | 13,25 |
| 17,6782 | 5,01714 | 100 |
| 19,8178 | 4,48005 | 27,73 |
| 21,2441 | 4,18238 | 14,42 |
| 21,8521 | 4,06737 | 7,02 |
| 22,3357 | 3,98038 | 39,37 |
| 23,2889 | 3,81958 | 10,11 |
| 23,9313 | 3,71848 | 64,55 |
| 24,3882 | 3,64985 | 17,32 |

(suite)

| 2-Theta (°) exp. | d (Å) exp. | Intensité (%) |
|---|---|---|
| 25,1812 | 3,53668 | 5,33 |
| 27,5931 | 3,23278 | 5,39 |
| 29,6861 | 3,00945 | 7,02 |
| 30,7722 | 2,90566 | 7,71 |

Angles de Bragg 2 thêta (exprimés en °±0,2) caractéristiques du diagramme de diffraction X sur poudre : 14,47°, 17,68°, 19,82°, 22,33°, 23,93°.

*Point de fusion : 108-110°C*

**Exemple 2 : Co-cristal Agomélatine/Acide malonique (1/1)**

Une solution de 300 mg de *N*-[2-(7-Méthoxy-1-naphtyl)éthyl]acétamide dans 15 ml de tert-butanol est ajoutée lentement à une solution de 129 mg d'acide malonique dans 35 ml d'eau dans un ballon de 250 ml. Le milieu est agité 30 minutes puis la solution est congelée à -40°C et séchée à cette même température pendant 2 jours pour conduire au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en °±0,2), et d'intensité relative (exprimée en pourcentage par rapport à la raie la plus intense) :

| 2-Theta (°) exp. | d (Å) exp. | Intensité (%) |
|---|---|---|
| 7,8661 | 11,23971 | 16,84 |
| 10,4713 | 8,44846 | 46,94 |
| 11,9502 | 7,406 | 45,62 |
| 12,7824 | 6,92563 | 9,99 |
| 14,7848 | 5,99187 | 21,65 |
| 15,3432 | 5,77504 | 19,95 |
| 16,0487 | 5,52273 | 100 |
| 16,7983 | 5,27793 | 11,99 |
| 16,9715 | 5,22445 | 13,9 |
| 17,1267 | 5,17745 | 9,19 |
| 21,0784 | 4,21489 | 9,77 |
| 22,3247 | 3,98233 | 23,32 |
| 24,0567 | 3,69939 | 6,29 |
| 24,5022 | 3,63313 | 56,82 |
| 25,0477 | 3,55523 | 23,07 |
| 25,2424 | 3,52825 | 40,38 |
| 25,7892 | 3,45467 | 10,44 |
| 26,7244 | 3,33585 | 7,17 |
| 27,3793 | 3,25753 | 20,44 |
| 27,9097 | 3,19682 | 26,63 |
| 29,4500 | 3,03304 | 10,41 |

(suite)

| 2-Theta (°) exp. | d (Å) exp. | Intensité (%) |
|---|---|---|
| 34,0469 | 2,63332 | 5,16 |

Angles de Bragg 2 thêta (exprimés en ° ±0,2) caractéristiques du diagramme de diffraction X sur poudre: 10,47°, 11,95°, 14,78°, 16,05°, 22,32°, 24,50°, 25,05°, 25,24°, 27,38°, 27,91°.

*Point de fusion : 67-68°C*

**Exemple 3** : **Co-cristal Agomélatine/Acide parahydroxybenzoïque (2/1)**

1 g de *N*-[2-(7-Méthoxy-1-naphtyl)éthyl]acétamide et 283,8 mg d'acide parahydroxybenzoïque sont introduits dans une jarre en inox de 25 ml. Deux billes en acier inox de 12 mm de diamètre sont ajoutées et la jarre est fermée. 200 µl d'éther isopropylique sont ajoutés. Des vibrations d'une fréquence de 30 Hz sont appliquées pendant 60 minutes, pour conduire au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en ° ±0,2), et d'intensité relative (exprimée en pourcentage par rapport à la raie la plus intense) :

| 2-Theta (°) exp. | d (Å) exp. | Intensité (%) |
|---|---|---|
| 10,6835 | 8,28111 | 11,39 |
| 11,9471 | 7,40793 | 8,16 |
| 12,0698 | 7,33288 | 12,04 |
| 13,1596 | 6,72799 | 22,29 |
| 14,6189 | 6,05948 | 6,29 |
| 14,7754 | 5,99567 | 11,14 |
| 14,907 | 5,94301 | 43,41 |
| 15,1499 | 5,84827 | 14,08 |
| 16,7697 | 5,28686 | 7,17 |
| 17,08 | 5,19149 | 8,17 |
| 17,2378 | 5,14433 | 10,12 |
| 17,3731 | 5,10456 | 20,24 |
| 17,5783 | 5,04543 | 16,57 |
| 18,3905 | 4,82442 | 24,81 |
| 18,7565 | 4,73108 | 11,19 |
| 18,9282 | 4,68855 | 23,85 |
| 19,0366 | 4,6621 | 21,45 |
| 19,4137 | 4,57238 | 8,15 |
| 19,6471 | 4,5186 | 20,4 |
| 19,9637 | 4,44765 | 20,12 |
| 20,1044 | 4,41683 | 19,09 |
| 20,2539 | 4,38456 | 20,62 |
| 20,9205 | 4,24635 | 10,62 |
| 21,491 | 4,13489 | 100 |

(suite)

| 2-Theta (°) exp. | d (Å) exp. | Intensité (%) |
|---|---|---|
| 21,7733 | 4,08191 | 91,9 |
| 22,2831 | 3,98966 | 7,75 |
| 23,7997 | 3,73875 | 12,32 |
| 23,9912 | 3,70935 | 8,36 |
| 24,2112 | 3,67614 | 6,78 |
| 24,6151 | 3,61672 | 17,26 |
| 24,9976 | 3,56224 | 22,13 |
| 26,5573 | 3,35646 | 4,98 |
| 26,7447 | 3,33337 | 5,85 |
| 27,5321 | 3,2398 | 12,36 |
| 29,4497 | 3,03306 | 12,87 |

Angles de Bragg 2 thêta (exprimés en ° ± 0,2) caractéristiques du diagramme de diffraction X sur poudre: 13,16°, 14,91°, 17,37°, 18,39°, 18,93°, 19,04°, 19,65°, 19,96°, 20,25°, 21,49°, 25,00°.

*Point de fusion : 93-95°C*

**<u>Exemple 4</u> : Co-cristal Agomélatine/Acide parahydroxybenzoïque (1/2)**

1 g de *N*-(2-(7-Méthoxy-1-naphtyl)éthyl]acétamide et 1,14 g d'acide parahydroxy benzoïque sont introduits dans une jarre en inox de 25 ml avec 250 μl de diisopropyléther. Deux billes en acier inox de 12 mm de diamètre sont ajoutées et la jarre est fermée. Des vibrations d'une fréquence de 30 Hz sont appliquées pendant 120 minutes, pour conduire au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en ° ± 0,2), et d'intensité relative (exprimée en pourcentage par rapport à la raie la plus intense) :

| 2-Theta (°) exp. | d (Å) exp. | Intensité (%) |
|---|---|---|
| 6,9836 | 12,65784 | 17,63 |
| 8,4549 | 10,45823 | 6,16 |
| 9,4969 | 9,31293 | 34,61 |
| 12,2797 | 7,208 | 38,63 |
| 12,9651 | 6,82845 | 14,3 |
| 13,1503 | 6,7327 | 7,88 |
| 13,7866 | 6,42337 | 7,33 |
| 13,9951 | 6,32814 | 27,1 |
| 15,7604 | 5,62307 | 52,5 |
| 16,1791 | 5,4785 | 32,32 |
| 16,6241 | 5,33282 | 51,26 |
| 17,5572 | 5,05145 | 39,19 |
| 18,1485 | 4,8882 | 54,91 |
| 18,3819 | 4,82664 | 17,31 |

(suite)

| 2-Theta (°) exp. | d (Å) exp. | Intensité (%) |
|---|---|---|
| 19,3253 | 4,5931 | 17,44 |
| 19,4415 | 4,56592 | 17,76 |
| 19,7593 | 4,49317 | 51,9 |
| 19,959 | 4,44867 | 42,09 |
| 21,0028 | 4,22989 | 45,52 |
| 21,2989 | 4,17175 | 20,42 |
| 22,0032 | 4,03979 | 60,83 |
| 22,6859 | 3,91973 | 11,33 |
| 22,9715 | 3,87164 | 20,19 |
| 23,5476 | 3,77821 | 39,55 |
| 23,7609 | 3,74477 | 93,42 |
| 24,4422 | 3,64191 | 32,21 |
| 25,3271 | 3,51664 | 19,07 |
| 25,5471 | 3,48685 | 14,62 |
| 26,0938 | 3,41502 | 100 |
| 26,8242 | 3,32367 | 21,88 |
| 26,9813 | 3,30467 | 16,4 |
| 27,9183 | 3,19586 | 6,85 |
| 28,4188 | 3,1407 | 27,49 |
| 28,7129 | 3,1092 | 30,36 |
| 29,276 | 3,05067 | 5,22 |
| 29,8536 | 2,99295 | 28,73 |
| 30,7825 | 2,90472 | 6,33 |
| 34,5702 | 2,59464 | 5,06 |

Angles de Bragg 2 thêta (exprimés en °±0,2) caractéristiques du diagramme de diffraction X sur poudre: 9,50°, 12,28°, 14,00°, 15,76°, 16,18°, 16,62°, 17,56°, 18,15°, 19,96°, 21,00°, 21,30°, 22,00°, 22,97°, 23,55°, 23,76°, 24,44°, 26,09°, 26,82°, 28,42°, 28,71°, 29,85°.

*Point de fusion : 116-118°C*

**<u>Exemple 5</u> : Co-cristal Agomélatine/Acide glutarique (1/1)**

1 g de *N*-[2-(7-Méthoxy-1-naphtyl)éthyl]acétamide et 555 mg d'acide glutarique sont introduits dans une jarre en inox de 25 ml. Deux billes en acier inox de 12 mm de diamètre sont ajoutées et la jarre est fermée. Des vibrations d'une fréquence de 30 Hz sont appliquées pendant 60 minutes, pour conduire au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en °±0,2), et d'intensité relative (exprimée en pourcentage par rapport à la raie la plus intense) :

| 2-Theta (°) exp. | d (Å) exp. | Intensité (%) |
|---|---|---|
| 9,5919 | 9,22091 | 22,85 |

(suite)

| 2-Theta (°) exp. | d (Å) exp. | Intensité (%) |
|---|---|---|
| 10,3486 | 8,5483 | 28,18 |
| 11,9618 | 7,39882 | 23,63 |
| 13,0927 | 6,76218 | 8,08 |
| 13,7395 | 6,44526 | 5,45 |
| 14,7283 | 6,0147 | 8,81 |
| 16,4376 | 5,39291 | 13,05 |
| 16,9847 | 5,2204 | 10,58 |
| 17,493 | 5,06987 | 10,05 |
| 17,6723 | 5,01881 | 6,83 |
| 18,6123 | 4,76741 | 17,35 |
| 18,9534 | 4,68238 | 15,44 |
| 19,9041 | 4,46083 | 16,48 |
| 20,5662 | 4,31869 | 20,46 |
| 21,6468 | 4,10548 | 38,05 |
| 21,9751 | 4,04488 | 5,01 |
| 22,0881 | 4,02444 | 5,94 |
| 23,3395 | 3,81143 | 100 |
| 23,7133 | 3,75217 | 6,65 |
| 24,0288 | 3,70362 | 5,71 |
| 24,6109 | 3,61733 | 5,25 |
| 25,0027 | 3,56152 | 6,82 |
| 25,863 | 3,44497 | 8,04 |
| 27,6684 | 3,22415 | 17,51 |
| 29,1279 | 3,06584 | 4,97 |

Angles de Bragg 2 thêta (exprimés en °±0,2) caractéristiques du diagramme de diffraction X sur poudre : 9,59°, 10,35°, 11,96°, 20,57°, 21,65°, 23,34°.

*Point de fusion : 74-75°C*

**Exemple 6 : Co-cristal Agomélatine/Acide cétoglutarique (1/1)**

1 g de *N*-[2-(7-Méthoxy-1-naphtyl)éthyl]acétamide et 600 mg d'acide cétoglutarique sont introduits dans une jarre en inox de 25 ml avec 500 µl d'éthanol. Deux billes en acier inox de 12 mm de diamètre sont ajoutées et la jarre est fermée. Des vibrations d'une fréquence de 30 Hz sont appliquées pendant 15 minutes, pour conduire après séchage pendant une nuit à 40°C, au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance inter-réticulaire d, d'angle de Bragg 2 thêta (exprimés en °±0,2), et d'intensité relative (exprimée en pourcentage par rapport à la raie la plus intense) :

| 2-Theta (°) exp. | d (Å) exp. | Intensité (%) |
|---|---|---|
| 5,2391 | 16,86816 | 18,25 |

(suite)

| 2-Theta (°) exp. | d (Å) exp. | Intensité (%) |
|---|---|---|
| 6,1796 | 14,30283 | 7,39 |
| 9,6513 | 9,16426 | 12,13 |
| 10,4827 | 8,43926 | 8,6 |
| 14,2638 | 6,20954 | 5 |
| 15,3616 | 5,76815 | 45,63 |
| 16,3452 | 5,41872 | 43,96 |
| 16,5381 | 5,35593 | 59,36 |
| 17,0478 | 5,20123 | 6,44 |
| 18,3191 | 4,84305 | 8,1 |
| 19,2396 | 4,61337 | 21,8 |
| 20,5617 | 4,31961 | 7,64 |
| 21,036 | 4,22329 | 12,12 |
| 21,3726 | 4,15752 | 7,66 |
| 23,57 | 3,77466 | 36,07 |
| 23,9026 | 3,7229 | 24,64 |
| 24,4145 | 3,64597 | 100 |
| 26,4474 | 3,37016 | 6,58 |
| 29,1314 | 3,06548 | 6,73 |
| 37,1969 | 2,41723 | 5,98 |

Angles de Bragg 2 thêta (exprimés en °±0,2) caractéristiques du diagramme de diffraction X sur poudre : 15,36°, 16,34°, 16,54°, 19,24°, 23,57°, 23,90°, 24,41°.

*Point de fusion : 94-96°C*

**Exemple 7 : Co-cristal Agomélatine/Acide glycolique (1/1)**

1 g de *N*-[2-(7-Méthoxy-1-naphtyl)éthyl]acétamide et 319 mg d'acide glycolique sont introduits dans une jarre en inox de 25 ml. Deux billes en acier inox de 12 mm de diamètre sont ajoutées et la jarre est fermée. Des vibrations d'une fréquence de 30 Hz sont appliquées pendant 15 minutes, pour conduire après séchage pendant une nuit à 40°C, au produit du titre qui est caractérisé par son point de fusion et par le diagramme de diffraction X sur poudre suivant, mesuré sur un diffractomètre Panalytical Xpert Pro MPD (anticathode de cuivre) et exprimé en termes de distance interréticulaire d, d'angle de Bragg 2 thêta (exprimés en °±0,2), et d'intensité relative (exprimée en pourcentage par rapport à la raie la plus intense) :

| 2-Theta (°) exp. | d (Å) exp. | Intensité (%) |
|---|---|---|
| 10,2906 | 8,59638 | 45,79 |
| 13,9365 | 6,35459 | 5,32 |
| 14,1139 | 6,27513 | 31,57 |
| 14,2265 | 6,22572 | 24,57 |
| 14,3625 | 6,16708 | 11,84 |
| 17,9846 | 4,93237 | 90,49 |

(suite)

| 2-Theta (°) exp. | d (Å) exp. | Intensité (%) |
|---|---|---|
| 18,617 | 4,76622 | 10,66 |
| 18,8288 | 4,71308 | 89,79 |
| 19,19 | 4,62519 | 9,61 |
| 19,5137 | 4,54918 | 30,43 |
| 19,941 | 4,45266 | 6,52 |
| 20,6101 | 4,30959 | 66,27 |
| 20,9906 | 4,23232 | 8,23 |
| 22,8209 | 3,89685 | 6,31 |
| 23,6248 | 3,76604 | 5,61 |
| 23,9623 | 3,71375 | 26,41 |
| 24,2171 | 3,67524 | 17,2 |
| 24,3906 | 3,64949 | 100 |
| 26,4458 | 3,37037 | 27,5 |
| 28,1154 | 3,1739 | 29,75 |
| 28,4808 | 3,134 | 5,71 |
| 28,6849 | 3,11217 | 6,41 |
| 28,9288 | 3,08648 | 5,75 |
| 29,518 | 3,02621 | 29,2 |
| 32,2458 | 2,77386 | 14,35 |

Angles de Bragg 2 thêta (exprimés en °±0,2) caractéristiques du diagramme de diffraction X sur poudre: 10,29°, 14,11°, 14,23°, 17,98°, 18,83°, 19,51°, 20,61°, 23,96°, 24,39°, 26,44°, 28,11°, 29,52°.

*Point de fusion : 75-77°C*

**Exemple 8 : Mesure de la vitesse de dissolution des co-cristaux**

La mesure des vitesses de dissolution des co-cristaux obtenus est réalisée à l'aide d'un appareil d'analyse μDiss (pION) en milieu acide et neutre à 37°C sous une vitesse d'agitation de 700 rpm. Les résultats obtenus sont rassemblés dans les tableaux suivants et sont exprimés en pourcentages d'augmentation de la vitesse de dissolution du co-cristal comparée à la vitesse de dissolution obtenue pour l'agomélatine de forme II contenue dans la forme commercialisée Valdoxan® :

$$\% = \frac{(\text{Vitesse de Dissolution cocrystal}) - (\text{Vitesse de Dissolution Valdoxan})}{(\text{Vitesse de Dissolution Valdoxan})} \times 100$$

| | HCl 0,01 N | Tampon pH 6,8 |
|---|---|---|
| Composé de l'Exemple 1 | +37% | +29% |
| Composé de l'Exemple 3 | +97% | +89% |
| Composé de l'Exemple 4 | +19% | +46% |

Les résultats obtenus montrent une augmentation de la vitesse de dissolution des co-cristaux allant de 29% à 97% dans l'une au moins des deux conditions acide ou neutre testée.

| | HCl 0,01 N | Tampon pH 6,8 |
|---|---|---|
| Composé de l'Exemple 2 | -55% | -21% |
| Composé de l'Exemple 5 | -42% | -29% |
| Composé de l'Exemple 6 | -47% | -32% |
| Composé de l'Exemple 7 | -30% | -30% |

Les résultats obtenus montrent une diminution de la vitesse de dissolution des co-cristaux allant de 21 % à 55% dans l'une au moins des deux conditions acide ou neutre testée.

**<u>Exemple 9 :</u> Composition pharmaceutique à libération accélérée**

Formule de préparation pour 1000 comprimés dosés à 25 mg d'agomélatine:

| | |
|---|---|
| Composé de l'Exemple 3 | 50 g |
| Lactose monohydrate | 115 g |
| Stéarate de Magnésium | 2 g |
| Amidon de maïs | 33 g |
| Maltodextrines | 15 g |
| Silice colloïdale anhydre | 1 g |
| Amidon de maïs prégélatinisé type A | 9 g |

**<u>Exemple 10</u> : Composition pharmaceutique à libération retardée**

Formule de préparation pour 1000 comprimés dosés à 25 mg :

| | |
|---|---|
| Composé de l'Exemple 6 | 50 g |
| Lactose monohydrate | 100 g |
| Stéarate de Magnésium | 2 g |
| Povidone | 12 g |
| Silice colloïdale anhydre | 1 g |
| Hypromellose | 85 g |

## Revendications

**1.** Co-cristal d'agomélatine **caractérisé en ce qu'**il est constitué :

- d'agomélatine ou *N*-(2-(7-méthoxy-1-naphtyl)éthyl]acétamide de formule (I)

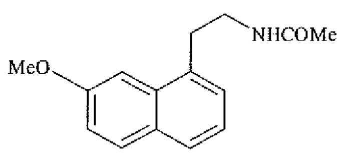

(I)

,

et
- d'un acide organique choisi parmi l'acide parahydroxybenzoïque, l'acide gallique, l'acide malonique, l'acide glutarique, l'acide glycolique, et l'acide cétoglutarique.

2. Co-cristal selon la revendication 1 **caractérisé en ce qu'**il modifie la vitesse de dissolution du principe actif par rapport à la forme cristalline II.

3. Co-cristal selon l'une des revendications 1 ou 2 **caractérisé en ce qu'**il confère une modification de la vitesse de dissolution du principe actif par rapport à la vitesse de dissolution de la forme cristalline II d'au moins 25% en condition neutre (pH 6,8) ou acide (HCl 0,01N).

4. Co-cristal selon l'une des revendications 1 à 3 qui est le *N*-[2-(7-méthoxy-1-naphtyl)éthyl]acétamide / acide parahydroxybenzoïque (2/1) caractérisé dans son diagramme de diffraction X sur poudre par les angles de Bragg 2 thêta (exprimés en °±0,2) 13,16°, 14,91°, 17,37°, 18,39°, 18,93°, 19,04°, 19,65°, 19,96°, 20,25°, 21,49°, 25,00°.

5. Co-cristal selon l'une des revendications 1 à 3 qui est le *N*-[2-(7-méthoxy-1-naphtyl)éthyl]acétamide / acide parahydroxybenzoïque (1/2) caractérisé dans son diagramme de diffraction X sur poudre par les angles de Bragg 2 thêta (exprimés en °±0,2) 9,50°, 12,28°, 14,00°, 15,76°, 16,18°, 16,62°, 17,56°, 18,15°, 19,96°, 21,00°, 21,30°, 22,00°, 22,97°, 23,55°, 23,76°, 24,44°, 26,09°, 26,82°, 28,42°, 28,71°, 29,85°.

6. Co-cristal selon l'une des revendications 1 à 3 qui est le *N*-[2-(7-méthoxy-1-naphtyl)éthyl]acétamide / acide gallique (2/1) caractérisé dans son diagramme de diffraction X sur poudre par les angles de Bragg 2 thêta (exprimés en °±0,2) 14,47°, 17,68°, 19,82°, 22,33°, 23,93°.

7. Co-cristal selon l'une des revendications 1 à 3 qui est le *N*-[2-(7-méthoxy-1-naphtyl)éthyl]acétamide / acide malonique (1/1) caractérisé dans son diagramme de diffraction X sur poudre par les angles de Bragg 2 thêta (exprimés en °±0,2) 10,47°, 11,95°, 14,78°, 16,05°, 22,32°, 24,50°, 25,05°, 25,24°, 27,38°, 27,91°.

8. Co-cristal selon l'une des revendications 1 à 3 qui est le *N*-[2-(7-méthoxy-1-naphtyl)éthyl] acétamide / acide glutarique (1/1) caractérisé dans son diagramme de diffraction X sur poudre par les angles de Bragg 2 thêta (exprimés en °±0,2) 9,59°, 10,35°, 11,96°, 20,57°, 21,65°, 23,34°.

9. Co-cristal selon l'une des revendications 1 à 3 qui est le *N*-[2-(7-méthoxy-1-naphtyl)éthyl] acétamide / acide glycolique (1/1) caractérisé dans son diagramme de diffraction X sur poudre par les angles de Bragg 2 thêta (exprimés en °±0,2) 10,29°, 14,11°, 14,23°, 17,98°, 18,83°, 19,51°, 20,61°, 23,96°, 24,39°, 26,44°, 28,11°, 29,52°.

10. Co-cristal selon l'une des revendications 1 à 3 qui est le *N*-[2-(7-méthoxy-1-naphtyl)éthyl] acétamide / acide cétoglutarique (1/1) caractérisé dans son diagramme de diffraction X sur poudre par les angles de Bragg 2 thêta (exprimés en °±0,2) 15,36°, 16,34°, 16,54°, 19,24°, 23,57°, 23,90°, 24,41 °.

11. Procédé d'obtention du co-cristal selon l'une des revendications 1 à 11 **caractérisé en ce que** :

   - les deux constituants sont mélangés au sein d'un solvant organique dans les proportions désirées (1 équivalent d'agomélatine pour 0,25 à 4 équivalents molaires d'acide organique) ;
   - la solution obtenue est agitée et optionnellement chauffée à une température au plus égale à la température d'ébullition du solvant choisi ;
   - le milieu est refroidi sous agitation et le co-cristal précipite naturellement ou précipite après reprise dans un deuxième solvant ;
   - le précipité obtenu est filtré et séché.

12. Procédé de préparation du co-cristal selon l'une des revendications 1 à 10 **caractérisé en ce que** les deux constituants sont co-broyés.

13. Procédé de préparation du co-cristal selon l'une des revendications 1 à 10 **caractérisé en ce que** les deux constituants sont mélangés au sein d'un solvant organique ou hydro-organique puis congelés et séchés entre -40°C et -60°C.

14. Procédé de préparation du co-cristal selon l'une des revendications 1 à 10 **caractérisé en ce que** les poudres d'agomélatine et de l'acide considéré sont mélangées dans un mélangeur, puis le mélange est extrudé par extrusion bi-vis sans filière pour obtenir un grain solide directement en sortie d'extrudeuse.

15. Compositions pharmaceutiques contenant comme principe actif un co-cristal selon l'une des revendications 1 à 10,

en combinaison avec un ou plusieurs véhicules inertes, non toxiques et pharmaceutiquement acceptables.

**16.** Compositions pharmaceutiques selon la revendication 15 utiles pour la fabrication de médicaments pour traiter les troubles du système mélatoninergique.

**17.** Compositions pharmaceutiques selon la revendication 15 utiles pour la fabrication de médicaments pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

**18.** Co-cristal selon l'une des revendications 1 à 10 pour le traitement des troubles du système mélatoninergique.

**19.** Co-cristal selon l'une des revendications 1 à 10 pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

## Patentansprüche

**1.** Co-Kristall von Agomelatin, **dadurch gekennzeichnet, dass** er gebildet ist aus:

- Agomelatin oder *N*-[2-(7-Methoxy-1-naphthyl)-ethyl]-acetamid der Formel (I)

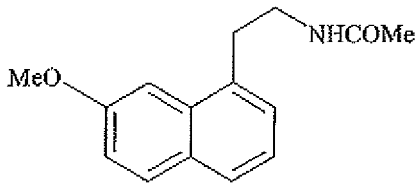

(I)

und

- einer organischen Säure ausgewählt aus para-Hydroxybenzoesäure, Gallussäure, Malonsäure, Glutarsäure, Glykolsäure und Ketoglutarsäure.

**2.** Co-Kristall nach Anspruch 1, **dadurch gekennzeichnet, dass** er die Lösungsgeschwindigkeit des Wirkstoffs, bezogen auf die Kristallform II, modifiziert.

**3.** Co-Kristall nach einem der Ansprüche 1 oder 2, **dadurch gekennzeichnet, dass** er dem Wirkstoff eine Modifizierung der Lösungsgeschwindigkeit, bezogen auf die Lösungsgeschwindigkeit der Kristallform II, um mindestens 25 % unter neutralen Bedingungen (pH 6,8) oder sauren Bedingungen (HCl, 0,01N) verleiht.

**4.** Co-Kristall nach einem der Ansprüche 1 bis 3, nämlich *N*-[2-(7-Methoxy-1-naphthyl)-ethyl]-acetamid / para-Hydroxybenzoesäure (2/1), **gekennzeichnet durch** die Bragg 2-Theta-Winkel (ausgedrückt in ° ±0,2) in seinem Pulverröntgenbeugungsdiagramm 13,16°, 14,91°, 17,37°, 18,39°, 18,93°, 19,04°, 19,65°, 19,96°, 20,25°, 21,49°, 25,00°.

5. Co-Kristall nach einem der Ansprüche 1 bis 3, nämlich *N*-[2-(7-Methoxy-1-naphthyl)-ethyl]-acetamid / para-Hydroxybenzoesäure (1/2), **gekennzeichnet durch** die Bragg 2-Theta-Winkel (ausgedrückt in ° ±0,2) in seinem Pulverröntgenbeugungsdiagramm 9,50°, 12,28°, 14,00°, 15,76°, 16,18°, 16,62°, 17,56°, 18,15°, 19,96°, 21,00°, 21,30°, 22,00°, 22,97°, 23,55°, 23,76°, 24,44°, 26,09°, 26,82°, 28,42°, 28,71°, 29,85°.

6. Co-Kristall nach einem der Ansprüche 1 bis 3, nämlich *N*-[2-(7-Methoxy-1-naphthyl)-ethyl]-acetamid / Gallussäure (211), **gekennzeichnet durch** die Bragg 2-Theta-Winkel (ausgedrückt in ° ±0,2) in seinem Pulverröntgenbeugungsdiagramm 14,47°, 17,68°, 19,82°, 22,33°, 23,93°.

7. Co-Kristall nach einem der Ansprüche 1 bis 3, nämlich *N*-[2-(7-Methoxy-1-naphthyl)-ethyl]-acetamid / Malonsäure (1/1), **gekennzeichnet durch** die Bragg 2-Theta-Winkel (ausgedrückt in ° ±0,2) in seinem Pulverröntgenbeugungsdiagramm 10,47°, 11,95°, 14,78°, 16,05°, 22,32°, 24,50°, 25,05°, 25,24°, 27,38°, 27,91°.

8. Co-Kristall nach einem der Ansprüche 1 bis 3, nämlich *N*-[2-(7-Methoxy-1-naphthyl)-ethyl]-acetamid / Glutarsäure (1/1), **gekennzeichnet durch** die Bragg 2-Theta-Winkel (ausgedrückt in ° ±0,2) in seinem Pulverröntgenbeugungsdiagramm 9,59°, 10,35°, 11,96°, 20,57°, 21,65°, 23,34°.

9. Co-Kristall nach einem der Ansprüche 1 bis 3, nämlich *N*-[2-(7-Methoxy-1-naphthyl)-ethyl]-acetamid / Glycolsäure (1/1), **gekennzeichnet durch** die Bragg 2-Theta-Winkel (ausgedrückt in ° ±0,2) in seinem Pulverröntgenbeugungsdiagramm 10,29°, 14,11°, 14,23°, 17,98°, 18,83°, 19,51°, 20,61°, 23,96°, 24,39°, 26,44°, 28,11°, 29,52°.

10. Co-Kristall nach einem der Ansprüche 1 bis 3, nämlich *N*-[2-(7-Methoxy-1-naphthyl)-ethyl]-acetamid / Ketoglutarsäure (1/1), **gekennzeichnet durch** die Bragg 2-Theta-Winkel (ausgedrückt in ° ±0,2) in seinem Pulverröntgenbeugungsdiagramm 15,36°, 16,34°, 16,54°, 19,24°, 23,57°, 23,90°, 24,41°.

11. Verfahren zur Herstellung des Co-Kristalls nach einem der Ansprüche 1 bis 10, **dadurch gekennzeichnet, dass** :

    - die beiden Bestandteile in den gewünschten Verhältnissen (1 Äquivalent Agomelatin für 0,25 bis 4 Moläquivalente der organischen Säure) in einem organischen Lösungsmittel vermischt werden;
    - die erhaltene Lösung gerührt und gegebenenfalls auf eine Temperatur erhitzt wird, die höchstens gleich ist der Siedetemperatur des gewählten Lösungsmittels;
    - das Medium unter Rühren abgekühlt wird und der Co-Kristall natürlich oder nach der Aufnahme in ein zweites Lösungsmittel ausfällt;
    - der erhaltene Niederschlag filtriert und getrocknet wird.

12. Verfahren zur Herstellung des Co-Kristalls nach einem der Ansprüche 1 bis 10, **dadurch gekennzeichnet, dass** die beiden Bestandteile gemeinsam vermahlen werden.

13. Verfahren zur Herstellung des Co-Kristalls nach einem der Ansprüche 1 bis 10, **dadurch gekennzeichnet, dass** die beiden Bestandteile in einem organischen oder wässrig-organischen Lösungsmittel vermischt und dann zwischen -40 °C und -60 °C gefriergetrocknet werden.

14. Verfahren zur Herstellung des Co-Kristalls nach einem der Ansprüche 1 bis 10, **dadurch gekennzeichnet, dass** die Pulver von Agomelatin und der betreffenden Säure in einem Mischer vermischt werden und die Mischung dann durch Doppelschnecken-extrusion ohne Mundstück extrudiert wird zur Bildung eines festen Korns direkt am Austritt des Extruders.

15. Pharmazeutische Zubereitungen enthaltend als Wirkstoff einen Co-Kristall nach einem der Ansprüche 1 bis 10 in Kombination mit einem oder mehreren inerten, nichttoxischen und pharmazeutisch annehmbaren Hilfsstoffen.

16. Pharmazeutische Zubereitungen nach Anspruch 15, nützlich für die Herstellung von Arzneimitteln zur Behandlung von Störungen des melatoninergischen Systems.

17. Pharmazeutische Zubereitungen nach Anspruch 15, nützlich für die Herstellung von Arzneimitteln zur Behandlung von Stress, Schlafstörungen, Angststörungen, und insbesondere allgemeinen Angststörungen, heftigen Obsessionsstörungen, Gemütsstörungen, und insbesondere bipolaren Störungen, von Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerzen, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Störungen der Gehirnzirkulation sowie von sexuellen Dysfunktionen, als Ovulationsinhibitoren, Immunomodulatoren und zur Behandlung von Krebserkrankungen.

**18.** Co-Kristall nach einem der Ansprüche 1 bis 10 zur Behandlung von Störungen des melatoninergischen Systems.

**19.** Co-Kristall nach einem der Ansprüche 1 bis 10 zur Behandlung von Stress, Schlafstörungen, Angststörungen, und insbesondere allgemeinen Angststörungen, heftigen Obsessionsstörungen, Gemütsstörungen, und insbesondere bipolaren Störungen, von Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerzen, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Störungen der Gehirnzirkulation sowie von sexuellen Dysfunktionen, als Ovulationsinhibitoren, Immunomodulatoren und zur Behandlung von Krebserkrankungen.

## Claims

**1.** Co-crystal of agomelatine, **characterised in that** it is composed of:

- agomelatine, or *N*-[2-(7-methoxy-1-naphthyl)ethyl]acetamide of formula (I)

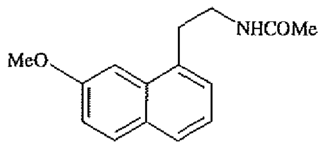

(I),

and
- an organic acid selected from para-hydroxybenzoic acid, gallic acid, malonic acid, glutaric acid, glycolic acid and ketoglutaric acid.

**2.** Co-crystal according to claim 1, **characterised in that** it modifies the active ingredient dissolution rate compared to the crystalline form II.

**3.** Co-crystal according to either claim 1 or claim 2, **characterised in that** it brings about a modification of the active ingredient dissolution rate compared to the dissolution rate of the crystalline form II of at least 25% under neutral (pH 6.8) or acid (0.01N HCl) conditions.

**4.** Co-crystal according to one of claims 1 to 3 which is *N*-[2-(7-methoxy-1-naphthyl)-ethyl]acetamide/para-hydroxybenzoic acid (2/1), **characterised in** its X-ray powder diffraction diagram by the Bragg's angles 2 theta (expressed in °±0.2) 13.16°, 14.91°, 17.37°, 18.39°, 18.93°, 19.04°, 19.65°, 19.96°, 20.25°, 21.49°, 25.00°.

**5.** Co-crystal according to one of claims 1 to 3 which is *N*-[2-(7-methoxy-1-naphthyl)-ethyl]acetamide/para-hydroxybenzoic acid (1/2), **characterised in** its X-ray powder diffraction diagram by the Bragg's angles 2 theta (expressed in °±0.2) 9.50°, 12.28°, 14.00°, 15.76°, 16.18°, 16.62°, 17.56°, 18.15°, 19.96°, 21.00°, 21.30°, 22.00°, 22.97°, 23.55°, 23.76°, 24.44°, 26.09°, 26.82°, 28.42°, 28.71°, 29.85°.

**6.** Co-crystal according to one of claims 1 to 3 which is *N*-[2-(7-methoxy-1-naphthyl)-ethyl]acetamide/gallic acid (2/1), **characterised in** its X-ray powder diffraction diagram by the Bragg's angles 2 theta (expressed in °±0.2) 14.47°, 17.68°, 19.82°, 22.33°, 23.93°.

**7.** Co-crystal according to one of claims 1 to 3 which is *N*-[2-(7-methoxy-1-naphthyl)-ethyl]acetamide/malonic acid (1/1), **characterised in** its X-ray powder diffraction diagram by the Bragg's angles 2 theta (expressed in °±0.2) 10.47°, 11.95°, 14.78°, 16.05°, 22.32°, 24.50°, 25.05°, 25.24°, 27.38°, 27.91°.

8. Co-crystal according to one of claims 1 to 3 which is *N*-[2-(7-methoxy-1-naphthyl)-ethyl]acetamide/glutaric acid (1/1), **characterised in** its X-ray powder diffraction diagram by the Bragg's angles 2 theta (expressed in °±0.2) 9.59°, 10.35°, 11.96°, 20.57°, 21.65°, 23.34°.

9. Co-crystal according to one of claims 1 to 3 which is *N*-[2-(7-methoxy-1-naphthyl)-ethyl]acetamide/glycolic acid (1/1), **characterised in** its X-ray powder diffraction diagram by the Bragg's angles 2 theta (expressed in °±0.2) 10.29°, 14.11°, 14.23°, 17.98°, 18.83°, 19.51°, 20.61°, 23.96°, 24.39°, 26.44°, 28.11°, 29.52°.

10. Co-crystal according to one of claims 1 to 3 which is *N*-[2-(7-methoxy-1-naphthyl)-ethyl]acetamide/ketoglutaric acid (1/1), **characterised in** its X-ray powder diffraction diagram by the Bragg's angles 2 theta (expressed in °±0.2) 15.36°, 16.34°, 16.54°, 19.24°, 23.57°, 23.90°, 24.41°.

11. Process for obtaining the co-crystal according to one of claims 1 to 10, **characterised in that**:

- the two constituents are mixed in an organic solvent in the desired proportions (1 equivalent of agomelatine per 0.25 to 4 molar equivalents of organic acid);
- the solution obtained is stirred and optionally heated at a temperature not greater than the boiling point of the selected solvent;
- the mixture is cooled, with stirring, and the co-crystal precipitates naturally or precipitates after taking up in a second solvent;
- the precipitate obtained is filtered and dried.

12. Process for the preparation of the co-crystal according to one of claims 1 to 10, **characterised in that** the two constituents are co-ground.

13. Process for the preparation of the co-crystal according to one of claims 1 to 10, **characterised in that** the two constituents are mixed in an organic or aqueous-organic solvent and then frozen and dried at from -40°C to -60°C.

14. Process for the preparation of the co-crystal according to one of claims 1 to 10, **characterised in that** powders of agomelatine and of the acid in question are mixed in a mixer and then the mixture is extruded by twin screw extrusion without a die in order to obtain a solid granular product directly at the extruder outlet.

15. Pharmaceutical compositions comprising as active ingredient a co-crystal according to one of claims 1 to 10, in combination with one or more pharmaceutically acceptable, inert, non-toxic carriers.

16. Pharmaceutical compositions according to claim 15 for use in the manufacture of medicaments for treating disorders of the melatoninergic system.

17. Pharmaceutical compositions according to claim 15 for use in the manufacture of medicaments for treating stress, sleep disorders, anxiety disorders and especially generalised anxiety disorder, obsessive-compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, and as ovulation inhibitors and immunomodulators and in the treatment of cancers.

18. Co-crystal according to one of claims 1 to 10 for the treatment of disorders of the melatoninergic system.

19. Co-crystal according to one of claims 1 to 10 for treating stress, sleep disorders, anxiety disorders and especially generalised anxiety disorder, obsessive-compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, and as ovulation inhibitors and immunomodulators and in the treatment of cancers.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0447285 A
- EP 1564202 A
- WO 200500256 A
- EP 1564202 A1


### Littérature non-brevet citée dans la description


- **SAI-LI ZHENG et al.** *Crystal Growth & Design,* 2011, vol. 11, 466-471